# EUROPEAN PATENT APPLICATION

(11) **EP 1 683 509 A1**
(43) Date of publication of application: **26.07.2006**
(21) Application number: 04799714.3
(22) Date of filing: 10.11.2004
(51) Int. Cl.: A61K 8/18

(54) **METHOD AND COMPOSITION FOR HAIR THICKENING**

(30) Priority: 11.11.2003 JP 2003381470
(71) Applicant: SHISEIDO COMPANY, LTD., Chuo-ku, Tokyo 104-8010 (JP)
(72) Inventor: EHAMA, Ritsuko, SHISEIDO RESEARCH CENTER, Yokohama-shi Kanagawa 2248558 (JP); IINO, Masato, SHISEIDO RESEARCH CENTER, Yokohama-shi Kanagawa 2248558 (JP); NAKAZAWA, Yosuke, SHISEIDO RESEARCH CENTER, Yokohama-shi Kanagawa 2248558 (JP); TAJIMA, Masahiro, SHISEIDO RESEARCH CENTER, Yokohama-shi Kanagawa 2248558 (JP); OGOU, Masashi, SHISEIDO RESEARCH CENTER, Yokohama-shi Kanagawa 2248558 (JP); ARASE, Seiji, Myouzai-gun Tokushima 7793213 (JP)
(74) Representative: Ulmann, Catherine Claire
(86) International application number: PCT/JP2004/017037
(87) International publication number: WO 2005/044205

(57) **Abstract**

The present invention provides a method for maintaining and promoting hair thickening by increasing the expression of keratinocyte growth factor (FGF-7) in hair follicle cells, and preferably dermal papilla cells, a composition for increasing expression of FGF-7 that contains adenosine and/or a derivative thereof, and particularly, an external scalp preparation for maintaining and promoting hair thickening.

SEQUENCE LISTING
<110> SHISEIDO COMPANY, LTD.
<120> Method and Composition for Thickening Hair
<130> P907
<150> JP2003-381470
<151> 2003-11-11
<160> 6

<210> 1
<211> 21
<212> DNA
<213> Artificial Sequence
<223> Forward Primer

<400> 1

catgaacacc cggagcacta c 21

<210> 2
<211> 25
<212> DNA
<213> Artificial Sequence
<223> Reverse Primer

<400> 2
cactgtgttc gacagaagag tcttc 25

<210> 3
<211> 22
<212> DNA
<213> Artificial Sequence
<223> Forward Primer

<400> 3
cacaaatgga tactgacatg ga 22

<210> 4
<211> 22
<212> DNA
<213> Artificial Sequence
<223> Reverse Primer

<400> 4
tcactcttat atcccctcct tc                                                22

<210> 5
<211> 24
<212> DNA
<213> Artificial Sequence
<223> Forward Primer

<400> 5
ctttgctcta cagatcatgc tttc                                              24

<210> 6
<211> 24
<212> DNA
<213> Artificial Sequence
<223> Reverse Primer

<400> 6
ttgccatagg aagaaagtgg gctg                                              24

## Description

### TECHNICAL FIELD

The present invention relates to a method for maintaining and promoting hair thickening by increasing expression of keratinocyte growth factor (FGF-7) in hair follicle cells, and preferably dermal papilla cells, a composition for increasing expression of FGF-7, and more particularly, to an external scalp preparation for maintaining and promoting hair thickening.

### BACKGROUND ART

In present day society with an increasing number of elderly and higher levels of stress, there are an increasing number of opportunities for hair being exposed to the risk of hair loss due to various causes. Various attempts are being made to provide better hair treatment preparations to accommodate this situation. Examples of the main effects offered by hair treatment preparations include: 1) hair growth induction effects (hair growth promotion effects, growth period induction effects), 2) effects that maintain hair thickness or increase hair thickness, namely, hair thickening effects, 3) hair growth period extending effects, 4) 5α-reductase inhibitory effects (early regression period transition inhibitory effects), 5) circulation promotion effects, 6) germicidal effects, 7) dandruff prevention effects, 8) moisturizing effects, 9) anti-oxidation effects, and the like.

Male pattern baldness is characterized by a reduction in the size of hair follicles caused by shortening of the hair growth period and a gradual decrease in hair diameter causing the hair to change to fine hair. Men with male pattern baldness have been shown to demonstrate extreme decreases in hair diameter even though they are hardly ever observed to demonstrate decreases in hair density (number of hairs per unit surface area) (Japanese Unexamined Patent Publication (Kokai) No. 2002-322094). On the basis of this fact, attention is being focused on not only induction of hair growth, but also on the importance of the aforementioned hair thickening effects.

However, what type of mechanism is involved in hair thickening has yet to be elucidated at the biochemical or molecular biological level. As a result, research and development of hair treatment preparations containing agents effective for maintaining and promoting hair thickening are still in the investigative stage. If the mechanism behind hair thickening is able to be adequately elucidated, it would be possible to provide hair treatment preparations having even more remarkable effects than existing hair treatment preparations.

### DISCLOSURE OF THE INVENTION

An object of the present invention is to elucidate the mechanism of hair thickening at the biochemical level, provide a method for maintaining and promoting hair thickening, and provide an external scalp composition for hair thickening.

FGF-7 is a glycoprotein composed of 194 amino acids, and is one of the growth factors belonging to the family of fibroblast growth factors. It is secreted by mesenchymal cells in the form of skin fibroblasts, and is well known to promote proliferation in a paracrine manner, by binding to specific FGFR2 IIIB receptors present in epidermal cells (Rubin, J.S. et al., Proc. Natl. Acad. Sci. USA 1989, 86: 802-806; Marchese C. et al., J. Cell Physiol. 1990, 144: 326-332). In addition, FGF-7 promotes proliferation of not only skin keratinocytes, but also a wide range of epithelial cells including liver mesenchymal cells and intestinal epithelial cells (Houseley, R.M. et al., J. Clin. Invest. 1994, 94: 1764-1777), and the proliferation of hair follicle keratinocytes is also known to be promoted by this FGF-7 (Pierce, G.F. et al., J. Exp. Med. 1994, 179: 831-840). In addition, experiments in mice have shown that FGF-7 is expressed in dermal papilla cells during the growth period, and the FGFR2 receptor essential for expression of its function has been shown to be expressed in hair matrix cells in the vicinity of dermal papilla cells (Dev. Dyn. 1996; 205(4): 379-386), thus suggesting the involvement of FGF-7 in hair growth. However, the role played by FGF-7 in hair growth has yet to be determined. Therefore, we hypothesized that increasing the expression of FGF-7 in dermal papilla cells ought to lead to hair thickening due to the action of extending the hair growth period by means of the proliferation of pilocytes which are considered to be its target cells.

First, when an attempt was made to find an agent that increases the expression of FGF-7 by allowing various agents to act on dermal papilla cells, it was determined that adenosine and derivatives thereof increase expression of FGF-7 gene. Next, when clinical tests were conducted on adenosine, it was surprisingly found to have hair thickening effects. As is described in Japanese Unexamined Patent Publication (Kokai) No. 2002-322094, since it is thought that at least three types of components consisting of components that demonstrate hair growth promotion effects by extending the hair growth period (e.g., sophora plant extracts), components that demonstrate hair loss prevention effects by inhibiting early transition to the regression period (e.g., testosterone), and components that demonstrate effects that induce transition from the dormant stage to the growth stage (e.g., decyltetradecyl aminoxide) are required to maintain and promote hair thickening, it is surprising to find that a single compound is able to demonstrate these effects.

Expression of FGF-7 in dermal papilla cells was found to be increased for the first time, and the finding that increases in the expression thereof is involved in hair thickening is a completely new discovery.

Thus, the present invention provides a method for maintaining and promoting hair thickening comprising increasing the expression of keratinocyte growth factor (FGF-7) in hair follicle cells, and preferably in dermal papilla cells.

Increased expression of FGF-7 in hair follicle cells is achieved by applying to the scalp an external skin preparation containing one or more types of agents that increase the expression of FGF-7 in hair follicle cells (to be referred to as an "FGF-7 expression accelerator") selected from the group consisting of adenosine and derivatives thereof such as adenosine 5'-phosphoric acid, adenosine 5'-phosphate, CCPA (2-chloro-N⁶⁻cyclopentyladenosine), C1-IB-MECA (2-chloro-N⁶-(3-iodobenzyl)-9-[5-(methylcarbamoyl)-β-D-ribofuranosyl]adenine) and NECA (N-ethylcarboxyamidoadenosine). Preferably, the agent is adenosine.

In a different aspect thereof, the present invention provides an FGF-7 expression increasing composition containing as an active component thereof an agent selected from the group consisting of adenosine and derivatives thereof such as adenosine 5'-phosphoric acid, adenosine 5'-phosphate, CCPA, C1-IB-MECA and NECA.

In a preferable mode thereof, expression of FGF-7 is increased in dermal papilla cells or outer root sheath cells.

In a preferable mode thereof, the agent is adenosine.

In a preferable mode thereof, the composition is an external skin preparation that maintains and promotes hair thickening by being applied to the scalp.

In another aspect thereof, the present invention provides a method for screening agents that maintain and promote hair thickening, comprising: applying a candidate agent to cells, preferably to hair follicle cells and more preferably to dermal papilla cells or outer root sheath cells, and selecting an agent that increases the expression of FGF-7 in said cells. Preferably, the dermal papilla cells are immortalized dermal papilla cells.

In a preferable mode thereof, increased expression of FGF-7 in the cells is determined by measuring the amount of FGF-7 in the cells.

In a more preferable mode thereof, the measurement is carried out by ELISA or RIA using a specific antibody to FGF-7.

In a more preferable mode thereof, increased expression of FGF-7 in the cells is determined by measuring the amount of mRNA that encodes FGF-7 extracted from the cells.

In a more preferable mode thereof, measurement of the mRNA is carried out by RT-PCR (reverse transcription-polymerase chain reaction).

The present invention is able to provide an effective method and composition for maintaining and promoting hair thickening.

### BRIEF DESCRIPTION OF THE DRAWINGS

Fig. 1 indicates the FGF-7 expression increasing effects of adenosine in dermal papilla cells in terms of the expressed amount of FGF-7 (relative value).
Fig. 2 indicates the FGF-7 expression increasing effects of adenosine in dermal papilla cells in terms of the relative amount of FGF-7 expressed 3 hours after addition of the agent.
Fig. 3 indicates the FGF-7 expression increasing effects of adenosine analogues in dermal papilla cells in terms of the relative amount of FGF-7 expressed 2 hours after addition of the agent (real-time PCR: n=4).
Fig. 4 indicates the FGF-7 expression increasing effects of flavanone in dermal papilla cells in terms of the relative amount of FGF-7 expressed 2 hours after addition of the agent (real-time PCR: n=4).
Fig. 5 indicates the FGF-7 expression increasing effects of 3,4'-dimethylflavanone in dermal papilla cells in terms of the relative amount of FGF-7 expressed 2 hours after addition of the agent (real-time PCR: n=3).
Fig. 6 indicates the FGF-7 expression increasing effects of 3-methylflavanone in dermal papilla cells in terms of the relative amount of FGF-7 expressed 2 hours after addition of the agent (real-time PCR: n=4).
Fig. 7 indicates the hair thickening effects of an adenosine-containing hair treatment preparation (thickening rate of 80 µm or more).
Fig. 8 indicates the FGF-7 expression increasing effects of adenosine in human immortalized dermal papilla cells in terms of the relative amount of FGF-7 expressed 2 hours after addition of the agent.
Fig. 9 indicates the FGF-7 expression increasing effects of adenosine in outer root sheath cells in terms of the relative amount of FGF-7 expressed 3 hours after addition of the agent.

### BEST MODE FOR CARRYING OUT THE INVENTION

The following provides an explanation of embodiments of the present invention.

The present invention provides a method for maintaining and promoting hair thickening by increasing the expression of keratinocyte growth factor (FGF-7) in hair follicle cells, and preferably dermal papilla cells.

Hair thickening in the present invention typically refers to inhibition of hair thinning caused by reduction in the size of hair roots, and the maintaining or increasing of hair thickness. Although it is preferable to assess the presence or absence of hair thickening effects on an individual basis since there are individual differences in hair thickness and quality, in general, as a result of continuously applying the FGF-7 expression promoter as claimed in the present invention for a period of, for example, 1 month, 3 months or 6 months or more, in an area on the head having a predetermined surface area such as 1 cm², 2 cm² or 5 cm², in the case the number of hairs having a predetermined diameter such as 40 µm, 60 µm, 80 µm or 100 µm or more has substantially not decreased, such as in the case the rate of decrease thereof is less than 10%, and preferably less than 5%, hair thickening is judged to be "maintained" and, hair in the case the number of hairs having a diameter of, for example, 40 µm, 60 µm, 80 µm or 100 µm or more has substantially increased, such as in the case the rate of increase is 5% or more, preferably 10% or more, and more preferably 20% or more, thickening is judged to have been promoted.

Increased expression of FGF-7 in hair follicle cells, and preferably dermal papilla cells, is achieved by applying to the scalp an external skin preparation containing one or more types of agents that increase expression of FGF-7 in hair follicle cells selected from the group consisting of adenosine and derivatives thereof such as adenosine 5'-phosphoric acid, adenosine 5'-phosphate, CCPA, C1-IB-MECA and NECA.

Adenosine is a kind of ribonucleoside that contains a purine derivative in the form of adenine in its nucleotide portion. Adenosine 5'-phosphoric acid is also referred to as 5'-adenylic acid, and is a nucleotide in which one molecule of phosphoric acid is bound to a hydroxyl group at the 5' position of adenosine ribose.

In addition, any substance may be used for a salt of adenosine 5'-phosphoric acid provided that it is a substance that forms an acid and a counter ion for the counter ion that forms the salt, examples of which include sodium, potassium and calcium. In addition, a hydrate thereof can also be used as a salt of adenosine 5'-phosphoric acid.

CCPA, C1-IB-MECA and NECA are adenosine analogues. CCPA, C1-IB-MECA and NECA can be acquired from Sigma Corporation.

Commercially available reagents can also be used for the aforementioned adenosine, adenosine 5'-phosphoric acid, adenosine 5'-phosphate, CCPA, C1-IB-MECA and NECA.

The FGF-7 expression increasing composition as claimed in the present invention may be an external skin preparation, and preferably an external scalp preparation such as a hair growth preparation or hair treatment preparation.

The FGF-7 expression increasing composition as claimed in the present invention contains, for example, 0.01 to 20.0% by weight, and preferably 0.1 to 10.0% by weight of the aforementioned FGF-7 expression accelerator based on the total amount of the composition. If the incorporated amount is less than 0.01% by weight of the total amount of the composition, thickening effects produced by the aforementioned component are not adequately demonstrated, thereby making this undesirable. In addition, if the incorporated amount exceeds 20.0% by weight, the tendency to cause problems during formulation becomes significant, thereby making this undesirable in certain cases.

The FGF-7 expression increasing composition as claimed in the present invention, and particularly the external skin application, can be administered transcutaneously by coating or spraying directly on the skin. In addition, although the dosage cannot be definitively specified since it varies according to the specific form of the external preparation, the age and symptoms of the user and so forth, in the case of administration to humans, the dosage is such that the FGF-7 expression accelerator is typically administered at 0.01 to 100.0 mg, and preferably 0.1 to 10.0 mg, per day per kilogram of body weight, and this dosage is preferably administered once a day, or two to four times per day.

The FGF-7 expression increasing composition as claimed in the present invention, and particularly the external skin preparation, demonstrates superior action that maintains and promotes hair thickening in humans and other mammals, and is useful in pharmaceuticals, over-the-counter pharmaceuticals and cosmetics for hair care.

The dosage forms that can be adopted by the FGF-7 expression increasing composition as claimed in the present invention, and particularly the skin external preparation, is preferably a dosage form of external use that can be applied to the outer skin, and can be selected from dosage forms such as liquids, milky lotions, creams and aerosols. In addition, the form of the composition of the present invention is also arbitrary, and examples of forms that can be adopted include tonics, hair creams, mousses, shampoos, rinses, milky lotions, beauty washes, facial packs and aerosols. The composition of the present invention is particularly preferably used by applying to the outer skin. There are no particular limitations on the method by which it is applied, and for example, a suitable amount such as 1 to 5 ml is preferably applied to the scalp corresponding to the skin surface area over which it is applied at least once a day, and for example, from 1 to 3 times per day.

In addition to the aforementioned fragrance contained as an essential component, the FGF-7 expression increasing composition a claimed in the present invention, and particularly the external skin preparation, may incorporate various types of oily and aqueous components, moisturizers, thickeners, preservatives, antioxidants, fragrances, colorants and various pharmaceuticals commonly used in cosmetics, over-the-counter pharmaceuticals and pharmaceuticals as necessary and within a range that does not impair the desired effects of the present invention.

For example, oily components such as higher fatty acids, solid paraffin, liquid paraffin, silicone oil, squalane, glyceryl monooleate, olive oil, isopropyl myristate, higher alcohols, or the like; moisturizers such as glycerin, hyaluronic acid, propylene glycol, maltitol, atherocollagen, sodium lactate, or the like; thickeners such as quince thickeners, carboxyvinyl polymers, xanthane rubber, or the like; vasodilators such as nicotinic acid amide, benzyl nicotinate, vitamin E acetate, swertia extract, carpronium chloride, acetylcholine derivatives, or the like; amino acids such as serine, methionine, arginine, or the like; vitamins such as vitamin B6, vitamin E, biotin, pantothenic acid, or the like; nicotinic acid esters such as nicotinic acid, methyl nicotinate, tocopherol nicotinate, or the like; skin function accelerators such as cepharanthine, or the like; female hormones such as estradiol, or the like; antiphlogistics such as glycyrrhizic acid, glycyrrhetinic acid, azulene, or the like; antimicrobials such as hinokitiol, hexachlorophene, benzalkonium chloride, cetyl pyridinium chloride, undecylenic acid, trichlorocarbanilide, bithionol, or the like; refreshing agents such as menthol, or the like; salicylic acid, zincs, lactic acid, or the like; and, organic acids such as citric acid, can be incorporated.

The FGF-7 expression increasing composition as claimed in the present invention, and particularly the external skin preparation, is recognized to additionally have hair growth action in addition to that of the aforementioned FGF-7 expression accelerator. More effective hair growth effects can be expected to be demonstrated by adding known hair growth components such as minoxidil and cyclosporine.

The present invention further provides a method for screening agents that maintain or promote hair thickening. This method is comprised of applying a candidate agent to cells, preferably hair follicle cells, and more preferably dermal papilla cells or outer root sheath cells, and selecting an agent that increases expression of FGF-7 in those cells.

Examples of dermal papilla cells that can be used include normal dermal papilla cells of human origin, and immortalized dermal papilla cells, which are advantageous in terms of being able to be easily acquired and having a rapid proliferation rate, such as immortalized human dermal papilla cells described in Japanese Unexamined Patent Publication (Kokai) No. 11-89565 that are obtained by transformation by SV40 large T antigen gene. In addition, mesenchymal cells that produce FGF-7, such as skin fibroblast cells isolated from humans or other cells originating in skin follicles such as outer root sheath cells, can also be used during screening in addition to the dermal papilla cells.

Increased expression of FGF-7 in cells is determined by, for example, measuring the amount of FGF-7 in the cells. Preferably, this measurement can be carried out by a method known among persons with ordinary skill in the art using a specific antibody to human FGF-7, examples of which include immunostaining methods using a fluorescent substance, pigment or enzyme, Western blotting, immunoassay methods such as ELISA or RIA, and various other methods. In addition, increased expression of FGF-7 in cells can also be determined by extracting RNA from the cells and measuring the amount of mRNA that encodes human FGF-7. Extraction of mRNA and measurement of the amount thereof is known among persons with ordinary skill in the art, and measurement of RNA is carried out by, for example, quantitative reverse transcription-polymerase chain reaction (RT-PCR). For example, quantitative PCR can be carried out using the combinations of primers indicated below.
Combination 1:
   Forward primer: 5'-CATGAACACCCGGAGCACTAC-3'
      (NM_002009:414-439) (SEQ. ID NO. 1)
   Reverse primer: 5'-CACTGTGTTCGACAGAAGAGTCTTC-3'
      (NM_002009:669-646) (SEQ. ID NO. 2)
   PCR product length: 251 bp
Combination 2:
   Forward primer: 5'-CACAAATGGATACTGACATGGA-3'
      (NM_002009:449-470) (SEQ. ID NO. 3)
   Reverse primer: 5'-TCACTCTTATATCCCCTCCTTC-3'
      (NM_002009:644-623) (SEQ. ID NO. 4)
   PCR product length: 196 bp (J Clin Endocrinol Metab 88(2), 773-, 2003)
Combination 3:
   Forward primer: 5'-CTTTGCTCTACAGATCATGCTTTC-3'
      (NM_002009:480-503) (SEQ. ID NO. 5)
   Reverse primer: 5'-TTGCCATAGGAAGAAAGTGGGCTG-3'
      (NM_002009:1022-999) (SEQ. ID NO. 6)
   PCR product length: 543 bp (J Clin Invest 92, 2408-, 1993)

Although the following provides a more detailed explanation of the present invention through its examples, it should not be interpreted that the technical scope of the present invention is limited by these examples. Furthermore, values used to represent incorporated amounts in the following examples represent the percent by weight based on the total weight of the product in which the amounts are incorporated unless specifically stated otherwise.

### Experiment 1 - Study of Increased Expression of FGF-7 by a Quantitative PCR Experiment (1)

### 1) Cell Culture

The human dermal papilla cells (DPC) used in the experiment consisted of DPC originating in a 34 year old woman that were placed in frozen storage after being isolated and cultured from human scalp provided as a byproduct of plastic surgery. The cells were disseminated to a cell density of 1.0 to 1.5 x 10⁴ cells/cm², and cultured in MEM (Gibco) containing 10% FBS under conditions of 37°C and 5% CO₂. The medium was replaced at the rate of twice per week, and the cells were harvested by separating from the dish with 0.25% trypsin when the cells had reached confluence (10 to 20 days after seeding) followed by disseminating again at a density of 1.0 - 1.5 x 10⁴ cells/cm².

### 2) Agent Treatment of Cultured Cells

After thawing and sub-culturing 1 to 3 times, the DPC were seeded into a 24-well plate at a density of 4.0 x 10⁴ cells/well, and the medium was replaced 4 days later with MEM (serum-free) in which adenosine had been dissolved to a concentration of 100 µM for the sub-confluence cells. Only MEM (serum-free) was used for the control cells, and these cells were treated in the same manner.

### 3) RT-PCR

mRNA was extracted from the cultured cells with MagNAPureLC (Roche Diagnostics) at 2, 4, 8 and 24 hours after addition of adenosine followed by cDNA synthesis using the SuperScriptII reverse transcriptase kit (Invitrogen). The expressed amounts of mRNA were compared by real-time PCR using the fluorescent pigment CyberGreen I, which binds to the minor grooves of doublestranded DNA, with the LightCycler-FastStart DNA Master SYBR Green I Kit (Roche Diagnostics) by using the synthesized cDNA as a template. More specifically, a total volume of 20 µl of reaction liquid (MgCl₂: 2 mM, forward and reverse primer: 0.25 µM each) was prepared according to the manual provided using the LightCycler-FastStart DNA Master SYBR Green I Kit (Roche Diagnostics), and a PCR reaction was carried out with the LightCycler (enzyme activation: 95°C/10 minutes; thermal denaturation: 95°C/15 seconds; annealing: 58°C/5 seconds; elongation reaction: 72°C/10 seconds; 40 cycles of thermal denaturation to elongation reaction) followed by monitoring fluorescent intensity at completion of the elongation reaction of each cycle. This fluorescent intensity reflects the amount of PCR product at that point in time. The amount of expressed gene was hypothesized to be that which is amplified while satisfying Y = A x 2^{x} for the PCR product Y of the initial amount of template A during the exponential amplification period of the PCR product relative to the number of PCR cycles X, and a relative value was calculated from the number of cycles needed to obtain a given amount of PCR product. The following indicates the FGF-7 amplification primers used in this study.
Forward primer: 5'-CATGAACACCCGGAGCACTAC-3'
(NM_002009:419-439) (SEQ. ID NO. 1)
Reverse primer: 5'-CACTGTGTTCGACAGAAGAGTCTTC-3'
(NM_002009:669-646) (SEQ. ID NO. 2)
PCR product size: 251 bp

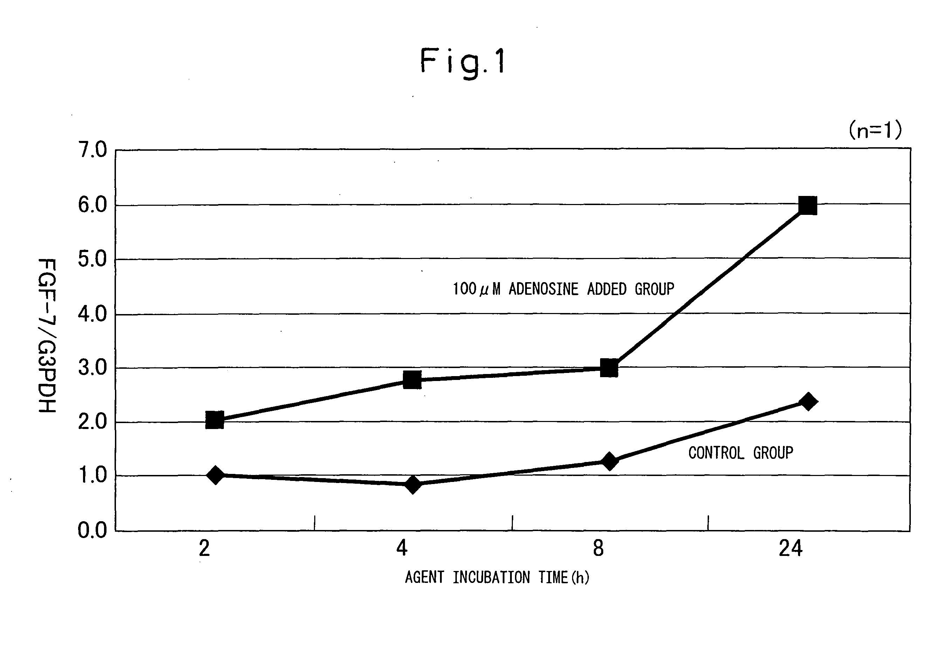

The results are shown in Fig. 1. As is clear from this figure, the expression of FGF-7 was observed to be increased considerably in dermal papilla cells treated with adenosine.

### Experiment 2 - Study of Increased Expression of FGF-7 by a Quantitative PCR Experiment (2)

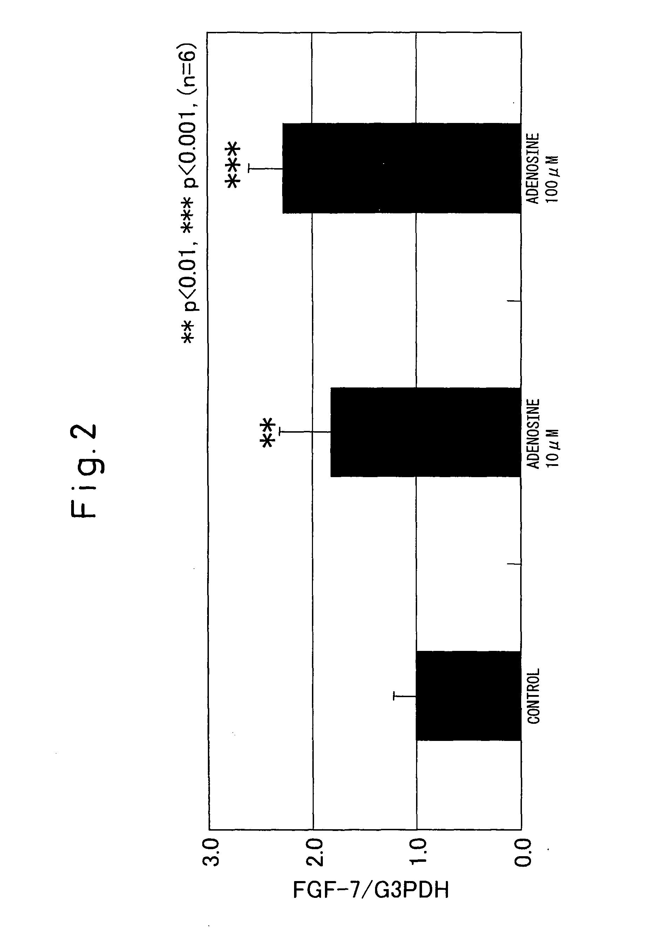

An RT-PCR experiment was carried out in the same manner as Experiment 1 with the exception of using two concentrations of adenosine consisting of 10 µM and 100 µM and setting the duration of adenosine treatment to 3 hours. The results are shown in Fig. 2. As is clear from this figure, expression of FGF-7 in dermal papilla cells treated with adenosine was confirmed to increase dependent on the concentration of adenosine.

### Experiment 3-1 - Study of Increased Expression of FGF-7 by a Quantitative PCR Experiment (3)

An RT-PCR experiment was carried out in the same manner as Experiment 1 with the exception of using the adenosine analogue CCPA (2-chloro-N⁶-cyclopentyladenosine) (100 µM), C1-IB-MECA (2-chloro-N⁶-(3-iodobenzyl)-9-[5-(methylcarbamoyl)-β-D-ribofuranosyl]adenine) (50 µM) or NECA (N-ethylcarboxyamidoadenosine) (10 µM) instead of adenosine, and setting the duration of agent treatment time to 3 hours. (Furthermore, in cases in which the solubility of the agent is low, all of the agent-containing media may be prepared to a DMSO concentration of 0.1%, including the agent-free control.)

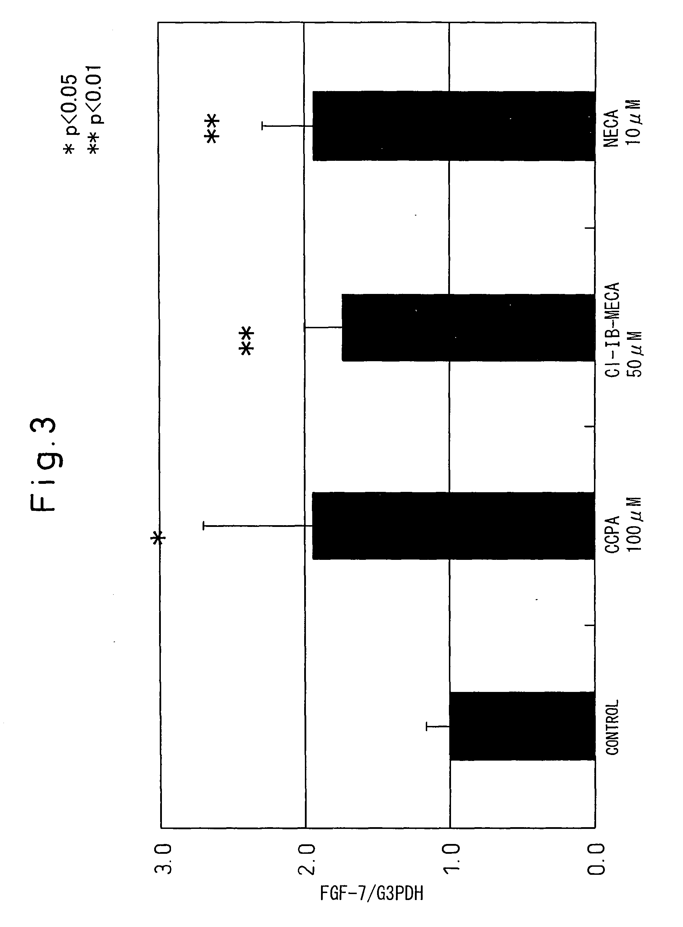

The results are shown in Fig. 3. As is clear from this figure, expression of FGF-7 in dermal papilla cells treated with CCPA, C1-IB-MECA or NECA was observed to be significantly increased.

### Experiment 3-2 - Study of Increased Expression of FGF-7 by a Quantitative PCR Experiment (4)

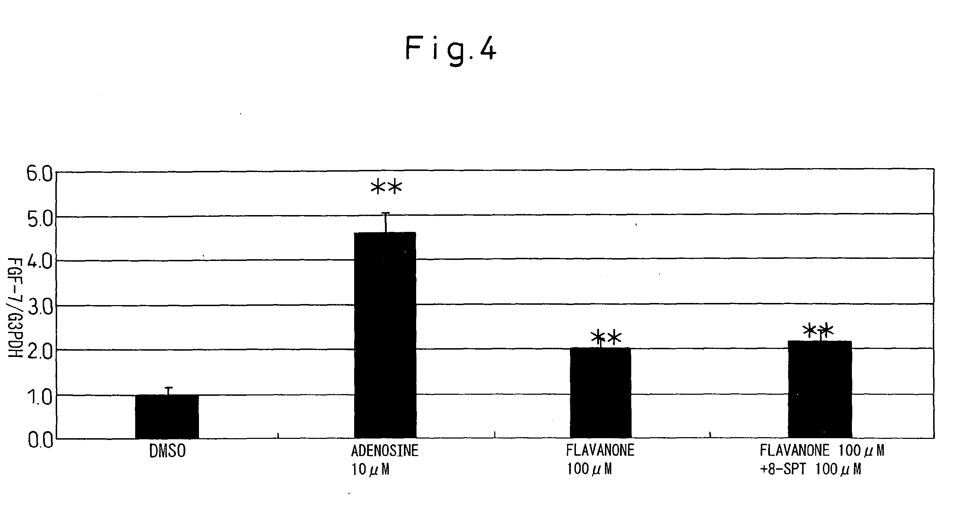

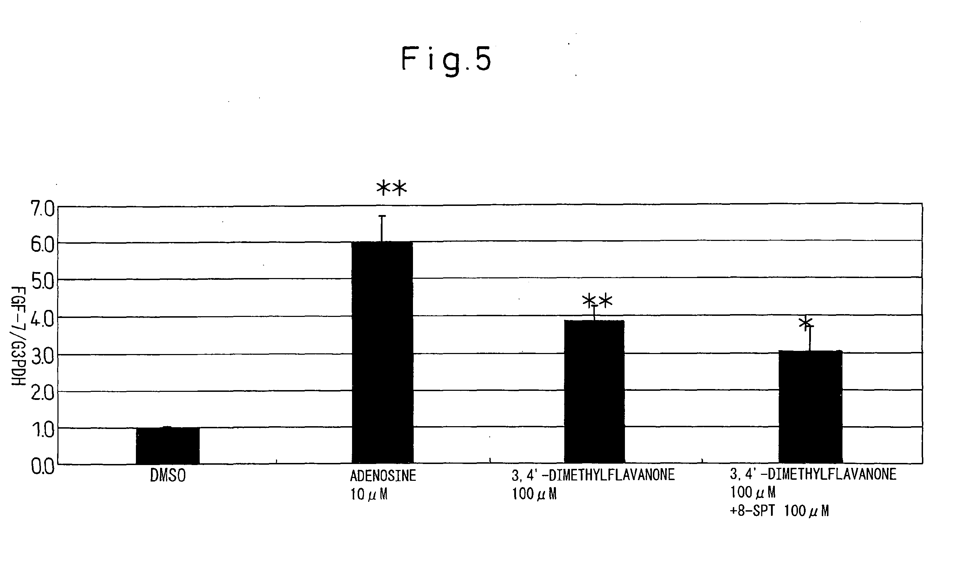

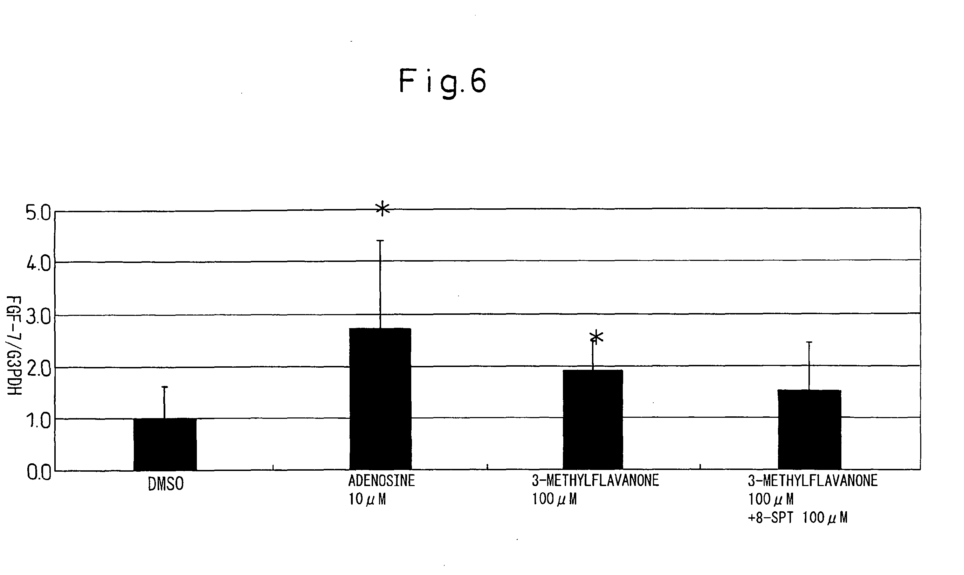

An RT-PCR experiment was carried out in the same manner as Experiment 1 with the exception of using flavanone, 3,4'-dimethylflavanone (YGU427) and 3-methylflavanone (YGU429) at concentrations of 100 µM each instead of adenosine, and setting the duration of agent treatment to 2 hours. The results are shown in Figs. 4, 5 and 6, respectively. Although results indicating increased expression of FGF-7 were obtained in each case, since inhibition by the adenosine receptor inhibitor, 8-SPT (8-sulfonyltheophylline) is not observed, this demonstrates that adenosine receptors are not necessarily required for increased expression of FGF-7.

### Experiment 4 - Study of Hair Thickening Effects of Adenosine on Hair

A study of hair thickening effects of adenosine among the various agents found to have the effect of increasing expression of FGF-7 as described above, was carried out according to the following method.

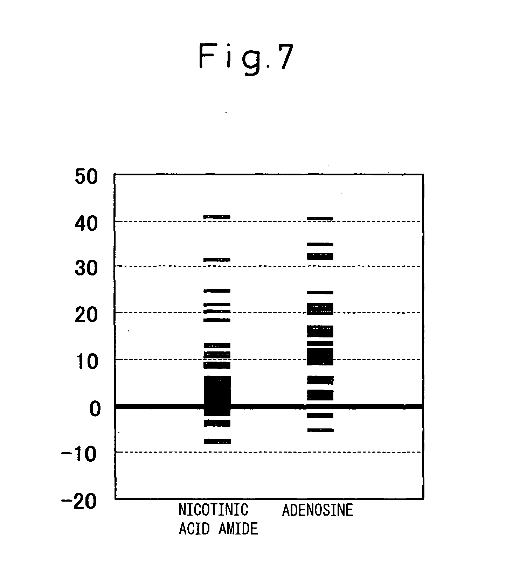

An adenosine-containing hair treatment preparation having the following Composition 1 and a nicotinic acid amide-containing hair treatment preparation having the following Composition 2 were applied to the scalps of male subjects age 30 to 50 years presenting with male pattern baldness (51 subjects in each group) in a suitable amount (roughly 2 to 3 ml) twice a day for six months followed by an investigation of the hair thickening effects of adenosine by comparing with hair thickening at the start of use. In this experiment, fine hair was defined as hair having a diameter of less than 40 µm, thick hair was defined as hair having a diameter of 60 µm or more, and remarkably thick hair was defined as hair having a diameter of 80 µm or more. At six months after using the adenosine-containing hair treatment preparation, fine hair decreased by 7% or more as compared with that at the start of the study, while thick hair having a diameter of 60 µm or more increased by 10% or more. Moreover, thick hair having a diameter of 80 µm or more increased by 5% or more. The continuous use of the adenosine-containing hair treatment preparation resulted in a prominent increase in thick hair as compared with the use of the nicotinic acid amide-containing hair treatment preparation. The results are shown in Fig. 7.

Furthermore, when the effects on hair density were investigated for the adenosine-containing hair treatment preparation and the nicotinic acid amide-containing hair treatment preparation were investigated, a statistically significant difference was not observed between the two (data not shown). Thus, adenosine, which demonstrates effects that increase expression of FGF-7 in cultured dermal papilla cells, was clearly demonstrated to be effective for maintaining or promoting hair thickness in particular.

**Adenosine-Containing Hair Treatment Preparation (Composition 1)**

| Component | Incorporated Amount (wt%) |
|---|---|
| Adenosine | 0.75 |
| Isostearyl alcohol | 0.50 |
| Polyoxyethyelene hydrogenated castor oil | 0.50 |
| Vinylpyrrolidone-N,N-dimethyl ethyl Methacrylate copolymer diethyl sulfate | 0.50 |
| Dipropylene glycol | 10.0 |
| Ethanol | 50.0 |
| Purified water | Remainder |
| DL-malic acid | As suitable |

**Nicotinic Acid Amide-containing Hair Treatment Preparation (Composition 2)**

| Component | Incorporated Amount (wt%) |
|---|---|
| Nicotinic acid amide | 0.10 |
| Isostearyl alcohol | 0.50 |
| Polyoxyethylene hydrogenated castor oil | 0.50 |
| Vinylpyrrolidone-N,N-dimethyl ethyl Methacrylate copolymer diethyl sulfate | 0.50 |
| Dipropylene glycol | 10.0 |
| Ethanol | 50.0 |
| Purified water | Remainder |
| DL-malic acid | As suitable |

### Experiment 5 - Study of Increased Expression of FGF-7 by a Quantitative PCR Experiment (4)

An RT-PCR experiment was carried out in the same manner as Experiment 1 with the exception of using human immortalized dermal papilla cells (immortalized human dermal papilla cells obtained by transformation by SV40 large T antigen gene described in Japanese Unexamined Patent Publication (Kokai) No. 11-89565) for the DPC, and setting the incubation time of agent treatment time to 2 hours.

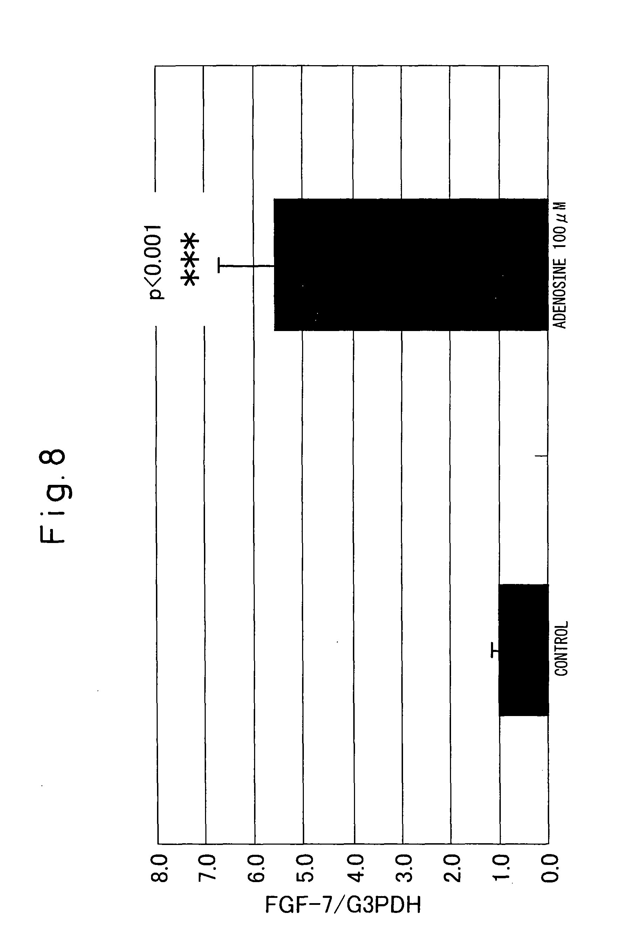

The results are shown in Fig. 8. As is clear from this figure, increased expression of FGF-7 by adenosine was also confirmed even when using human immortalized dermal papilla cells instead of the dermal papilla cells. Thus, it was clearly demonstrated that human immortalized dermal papilla cells can also be used in screening for FGF-7 expression accelerators.

### Experiment 6 - Study of Increased Expression of FGF-7 by a Quantitative PCR Experiment (5)

### 1) Cell Culture

The human outer root sheath cells (ORS) used in the experiment consisted of ORS originating in a 40 year old woman that were placed in frozen storage after being isolated and cultured from human scalp provided as a byproduct of plastic surgery. The cells were seeded to a cell density of 1.0 to 1.5 x 10⁴ cells/cm², and cultured in K-SFM medium (Gibco) under conditions of 37°C and 5% CO₂. The ORS cells at P3 were seeded in a 24-well plate at a density of 2.0 x 10⁴ cells/well, and the medium was replaced three days later with KBM medium (Kurabo) in which adenosine had been dissolved to a concentration of 10 or 100 µM for the sub-confluence cells. Only KBM medium was used for the control cells, and the control cells were treated in the same manner. RT-PCR was carried out in the same manner as Experiment 1.

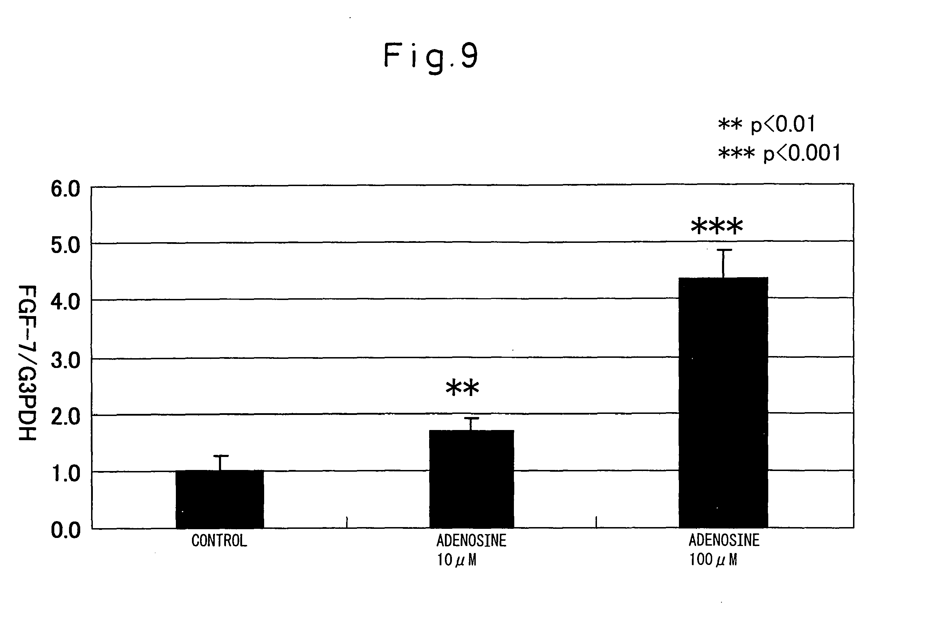

The results are shown in Fig. 9. As is clear from this figure, a prominent increase in expression of FGF-7 was also observed in outer root sheath cells treated with adenosine in the same manner as with dermal papilla cells.

### INDUSTRIAL APPLICABILITY

The present invention makes it possible to provide a method and composition that are effective in maintaining or promoting hair thickening.

## Claims

1. A method for maintaining and promoting hair thickening comprising increasing the expression of keratinocyte growth factor (FGF-7) in hair follicle cells.

2. A method according to claim 1, wherein expression of the FGF-7 is increased by applying to the scalp an external skin preparation containing one or more types of agents that increase the expression of FGF-7 in hair follicle cells selected from the group consisting of adenosine, adenosine 5'-phosphoric acid, adenosine 5'-phosphate, CCPA (2-chloro-N⁶-cyclopentyladenosine), C1-IB-MECA (2-chloro-N⁶-(3-iodobenzyl)-9-[5-(methylcarbamoyl)-β-D-ribofuranosyl]adenine) and NECA (N-ethylcarboxyamidoadenosine).

3. A method according to claim 2, wherein at least one type of the agent that increases expression of FGF-7 in the hair follicle cells is adenosine.

4. A method according to any of claims 1 to 3, wherein the hair follicle cells are dermal papilla cells or outer root sheath cells.

5. A composition for increasing expression of FGF-7 comprising as an active component thereof an agent selected from the group consisting of adenosine, adenosine 5'-phosphoric acid, adenosine 5'-phosphate, CCPA, C1-IB-MECA and NECA.

6. A composition according to claim 5, wherein at least one type of the agents is adenosine.

7. A composition according to claim 5 or 6 that is an external skin preparation that maintains and promotes hair thickening by being applied to the scalp.

8. A method for screening agents that maintain and promote hair thickening, comprising: applying a candidate agent to cells, and selecting an agent that increases the expression of FGF-7 in said cells.

9. A method according to claim 8, wherein increased expression of FGF-7 in the cells is determined by measuring the amount of mRNA that encodes FGF-7 extracted from the cells.

10. A method according to claim 8 or 9, wherein the cells are dermal papilla cells, immortalized dermal papilla cells or outer root sheath cells.
